# EUROPEAN PATENT APPLICATION

(11) **EP 1 374 868 A1**
(43) Date of publication of application: **02.01.2004**
(21) Application number: 02702893.5
(22) Date of filing: 12.03.2002
(51) Int. Cl.: A61K 31/495, A61K 31/551, A61K 31/4164, A61K 31/395, A61P 9/10, A61P 3/10, C07D 295/08

(54) **REMEDIES AND/OR PREVENTIVES FOR DIABETIC ISCHEMIC HEART DISEASES**

(30) Priority: 13.03.2001 JP 2001069552; 13.03.2001 JP 2001069553
(71) Applicant: Mitsubishi Pharma Corporation, Osaka-shi, Osaka 541-0046 (JP)
(72) Inventor: KITADA, Yoshimi, MITSUBISHI PHARMA CORPORATION, Chuo-ku, Tokyo 103-8405 (JP); SATOH, Naoya, MITSUBISHI PHARMA CORPORATION, Chuo-ku, Tokyo 103-8405 (JP)
(74) Representative: HOFFMANN - EITLE
(86) International application number: PCT/JP2002/002285
(87) International publication number: WO 2002/072097

(57) **Abstract**

An agent for therapeutic and /or prophylactic treatment of heart failure or arrhythmia in diabetic ischemic heart disease, which comprises as an active ingredient an aminobenzenesulfonic acid derivative represented by the following general formula (I): 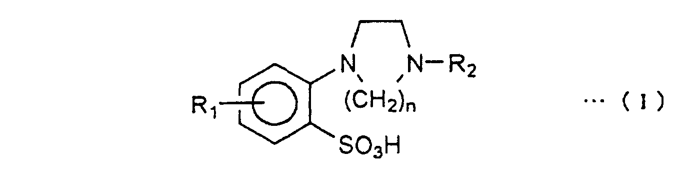 (wherein R₁ represents, for example, hydrogen atom; R₂ represents, for example, hydrogen atom; and n represents an integer of from 1 to 4) or a salt thereof, or a hydrate thereof or a solvate thereof, and an agent for therapeutic and /or prophylactic treatment of diabetic ischemic heart disease wherein said agent improves a cardiac dysfunction in diabetic ischemic heart disease.

## Description

### Technical Field

The present invention relates to an agent for therapeutic and /or prophylactic treatment of diabetic ischemic heart disease. More specifically, the present invention relates to an agent for therapeutic and /or prophylactic treatment of heart failure or arrhythmia in diabetic ischemic heart disease, which comprises as an active ingredient a specific aminobenzenesulfonic acid derivative or a salt thereof, or a hydrate thereof or a solvate thereof, and an agent for therapeutic and /or prophylactic treatment of a diabetic ischemic heart disease which is characterized to improve a cardiac dysfunction in diabetic ischemic heart disease.

### Background Art

It is known that cardiac myocytes are damaged along progress of diabetes, which later leads to cardiomyopathy or heart failure. A leak of calcium ion from sarcoplasmic reticulum in a cardiac myocyte arises to increase a concentration of free calcium ion to induce a systolic and diastolic dysfunction or arrhythmia is considered to be a cause of the damage. Therefore, a medicament which inhibits the leak of calcium ion from sarcoplasmic reticulum is expected to have a high therapeutic effect on heart failure or arrhythmia in diabetic cardiomyopathy. However, no medicament which directly inhibits a leak of calcium ion from sarcoplasmic reticulum has been known so far.

Resistances against acidosis due to ischemia or the like is significantly decreased in patients with diabetes. For example, when patients with diabetes have ischemic heart disease such as myocardial infarction, mortality significantly increases because of severe heart failure. An exacerbated cardiac dysfunction due to decreased action of calcium ion on contractile protein system caused by acidosis is considered to be a cause of decreased resistance. For a therapeutic treatment of the aforementioned disease, positive inotropic effect by cardiotonic agents and a cardiac protection by ACE inhibitors have been expected to be useful. However, an improvement of contractility by cardiotonic agents in the acidosis was not so effective as expected. In addition, improvement of cardiac function by ACE inhibitors has not been sufficient, because the effect of ACE inhibitors is not improvement of cardiac function but secondary to cardiac protection. Therefore, a medicament for improving a cardiac dysfunction in diabetic ischemic heart disease, which is resistant to acidosis, has been desired.

Aminobenzenesulfonic acid derivatives, which have inhibitory action on excessive accumulation of intracellular calcium ions in cardiac muscle and vascular smooth muscle, are known (Japanese Patent Unexamined Publication (KOKAI) No. Hei 3-7263). As for these compounds, publications disclose that they inhibit or reduce a myocardial injury, cardiac conduction system disorder and the like, without a *β* receptor stimulator-like action, a *β* receptor antagonist-like action, a calcium channel antagonist-like action, and thus they could be potential agents useful for prophylactic or therapeutic treatment for ischemic heart disease, heart failure, hypertension, arrhythmia, and the like (Japanese Patent Unexamined Publication (KOKAI) No. Hei 3-7263 and Japanese Patent Unexamined Publication (KOKAI) No. Hei 4-139127). Japanese Patent Unexamined Publication (KOKAI) No. Hei 10-298077 discloses that the aforementioned compounds have an action of significantly improving cardiac dysfunction under pathological condition of cardiomyopathy and improving long-term survival rate in idiopathic cardiomyopathy to achieve apothanasia; and WO99/40919 discloses that the aforementioned compounds have an action of enhancing calcium ion uptake into cardiac sarcoplasmic reticulum, and thus they are useful for therapeutic and/or prophylactic treatment of diastolic dysfunction.

However, these publications neither suggest nor instruct that the aforementioned compounds have an inhibitory action against the leak of calcium ion from sarcoplasmic reticulum in a diabetic ischemic heart disease, which has not been known for any conventional medicaments. Further, these publications neither suggest nor instruct that the aforementioned compound have an acidosis-resistant action of improving cardiac dysfunction in diabetic ischemic heart disease, which has not been known for any conventional medicaments.

An object of the present invention is to provide a medicament for improving cardiac dysfunction in a diabetic ischemic heart disease. More specifically, the object is to provide a medicament for acidosis-resistant improvement of cardiac dysfunction in diabetic ischemic heart disease. In addition, the object of the present invention is to provide a medicament for therapeutic and /or prophylactic treatment of heart failure or arrhythmia in a diabetic ischemic heart disease. More specifically, the object of the present invention is to provide a medicament which has an action of inhibiting a leak of calcium ion from sarcoplasmic reticulum in diabetic ischemic heart disease, and thus having a high therapeutic and /or prophylactic effect for heart failure or arrhythmia in diabetic ischemic heart disease.

### Disclosure of the Invention

The inventors of the present invention conducted various researches to achieve the foregoing objects, and as a result, they found that a particular class of aminobenzenesulfonic acid or a salt thereof, or a hydrate thereof or a solvate thereof has an inhibitory action against the leak of calcium ion from sarcoplasmic reticulum in a diabetic ischemic heart disease, and thus having a high therapeutic and /or prophylactic effect for heart failure or arrhythmia in diabetic ischemic heart disease. The present invention was achieved on the basis of these findings. In addition, they realized that, for an evaluation of the effect of acidosis on myocardial contraction, skinned fiber preparation was the best evaluation system, which enables assessment of direct action of calcium ions on a contractile protein system, and they evaluated the particular aminobenzenesulfonic acid derivative or a salt thereof, or a hydrate thereof or a solvate thereof by using the preparation. As a result, they found that the aforementioned compounds had acidosis-resistant action of improving cardiac dysfunction in diabetic ischemic heart disease, and they could be potential new-type medicaments for diabetic ischemic heart disease. The present invention was achieved on the basis of the above findings.

The gists of the present invention are as follows.
1. An agent for therapeutic and /or prophylactic treatment of heart failure or arrhythmia in diabetic ischemic heart disease, which comprises as an active ingredient an aminobenzenesulfonic acid derivative represented by the following general formula (I) 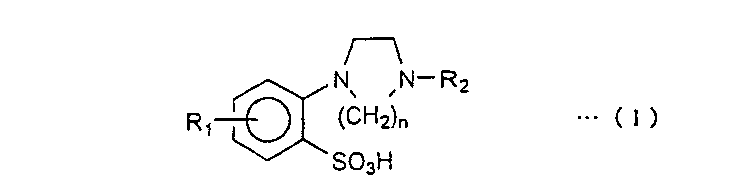 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.
2. The aforementioned agent for therapeutic and /or prophylactic treatment, characterized in that the diabetic ischemic heart disease is diabetic cardiomyopathy.
3. The aforementioned agent for therapeutic and /or prophylactic treatment, characterized in that said agent is based on an inhibition of a leak of calcium ion from sarcoplasmic reticulum in the diabetic ischemic heart disease.
4. An agent for therapeutic and /or prophylactic treatment of a disease caused by a leak of calcium ion from sarcoplasmic reticulum, which comprises as an active ingredient an aminobenzenesulfonic acid derivative represented by the following general formula (I) 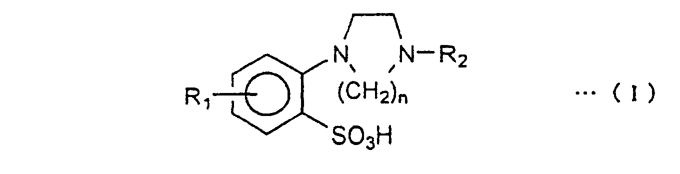 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.
5. An agent for therapeutic and /or prophylactic treatment of diabetic ischemic heart disease, characterized in that said agent improves a cardiac dysfunction in diabetic ischemic heart disease, and comprises as an active ingredient an aminobenzenesulfonic acid derivative represented by the following general formula (I) 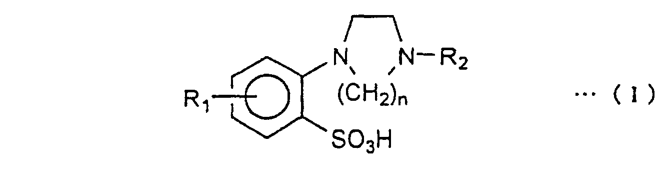 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.
6. The aforementioned agent for therapeutic and /or prophylactic treatment, characterized in that the diabetic ischemic heart disease is diabetic cardiomyopathy.
7. The aforementioned agent for therapeutic and /or prophylactic treatment, characterized in that the cardiac dysfunction is cardiac dysfunction resistant to acidosis.
8. An agent for therapeutic and /or prophylactic treatment of a disease caused by cardiac dysfunction resistant to acidosis, which comprises as an active ingredient an aminobenzenesulfonic acid derivative represented by the following general formula (I) 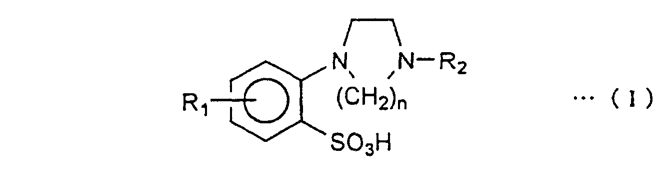 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.
9. Any one of the foregoing agents for therapeutic and /or prophylactic treatment, wherein the substituting position of R₁ is 5-position.
10. Any one of the foregoing agents for therapeutic and /or prophylactic treatment, wherein n is 2.
11. Any one of the foregoing agents for therapeutic and /or prophylactic treatment, wherein R₂ is hydrogen atom, a C₁-C₃ alkyl group, or a C₇-C₁₂ aralkyl group which may have one or more substituents selected from the group consisting of a C₁-C₃ alkyl group, a C₁-C₃ alkoxy group, and a halogen atom.
12. Any one of the foregoing agents for therapeutic and /or prophylactic treatment, wherein R₂ is hydrogen atom or a C₇-C₁₂ aralkyl group which may have one or more substituents selected from the group consisting of a C₁-C₃ alkoxy group.
13. Any one of the foregoing agents for therapeutic and /or prophylactic treatment, wherein R₂ is hydrogen atom.
14. Any one of the foregoing agents for therapeutic and /or prophylactic treatment, wherein R₁ is hydrogen atom, a C₁-C₆ alkyl group, a C₅-C₆ cycloalkyl group, trifluoromethyl group, a halogen atom, or phenyl group.
15. Any one of the foregoing agents for therapeutic and /or prophylactic treatment, wherein R₁ is a C₁-C₃ alkyl group, cyclohexyl group, trifluoromethyl group, chlorine atom, bromine atom, or phenyl group.
16. Any one of the foregoing agents for therapeutic and /or prophylactic treatment, wherein R₁ is methyl group or propyl group.
17. Any one of the foregoing agents for therapeutic and /or prophylactic treatment, wherein the active ingredient is selected from the following compounds:
   5-methyl-2-(1-piperazinyl) benzenesulfonic acid;
   5-trifluoromethyl-2-(1-piperazinyl) benzenesulfonic acid;
   5-n-propyl-2-(1-piperazinyl) benzenesulfonic acid;
   5-phenyl-2-(1-piperazinyl) benzenesulfonic acid;
   5-chloro-2-(1-piperazinyl) benzenesulfonic acid;
   5-bromo-2-(1-piperazinyl) benzenesulfonic acid;
   5-isopropyl-2-(1-piperazinyl) benzenesulfonic acid;
   5-cyclohexyl-2-(1-piperazinyl) benzenesulfonic acid;
   5-n-propyl-2-(1-homopiperazinyl) benzenesulfonic acid;
   5-n-propyl-2-[4-(2,3,4-trimethoxybenzyl)-1-piperazinyl] benzenesulfonic acid; and
   5-n-propyl-2-[4-(3,4-dimethoxybenzyl)-1-piperazinyl] benzenesulfonic acid.
18. Any one of the foregoing agents for therapeutic and /or prophylactic treatment, wherein the active ingredient is selected from the following compounds:
   5-methyl-2-(1-piperazinyl) benzenesulfonic acid; and
   5-n-propyl-2-(1-piperazinyl) benzenesulfonic acid.
19. Any one of the foregoing agents for therapeutic and /or prophylactic treatment, wherein the active ingredient is 5-methyl-2-(1-piperazinyl) benzenesulfonic acid monohydrate.
20. An inhibitor of a leak of calcium ion from sarcoplasmic reticulum in diabetic ischemic heart disease, which comprises as an active ingredient an aminobenzenesulfonic acid derivative represented by the following general formula (I) 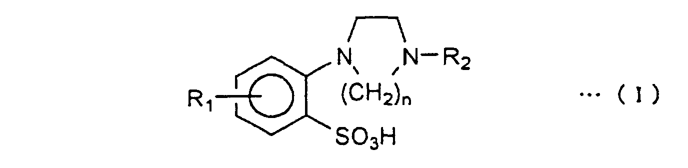 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.
21. An agent for acidosis-resistant improvement of cardiac dysfunction in diabetic ischemic heart disease, which comprises as an active ingredient an aminobenzenesulfonic acid derivative represented by the following general formula (I) 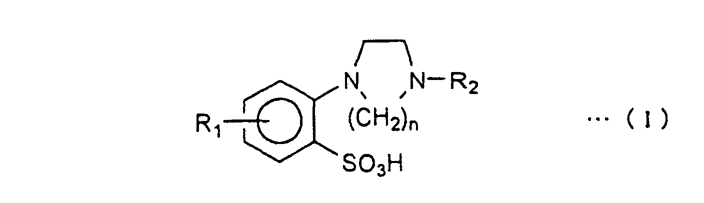 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.
22. The aforementioned medicament, wherein the substituting position of R₁ is 5-position.
23. Any one of the foregoing medicaments, wherein n is 2.
24. Any one of the foregoing medicaments, wherein R₂ is hydrogen atom, a C₁-C₃ alkyl group, or a C₇-C₁₂ aralkyl group which may have one or more substituents selected from the group consisting of a C₁-C₃ alkyl group, a C₁-C₃ alkoxy group, and a halogen atom.
25. Any one of the foregoing medicaments, wherein R₂ is hydrogen atom, or a C₇-C₁₂ aralkyl group which may have one or more substituents selected from the group consisting of a C₁-C₃ alkoxy group.
26. Any one of the foregoing medicaments, wherein R₂ is hydrogen atom.
27. Any one of the foregoing medicaments, wherein R₁ is hydrogen atom, a C₁-C₆ alkyl group, a C₅-C₆ cycloalkyl group, trifluoromethyl group, a halogen atom, or phenyl group,
28. Any one of the foregoing medicaments, wherein R₁ is a C₁-C₃ alkyl group, cyclohexyl group, trifluoromethyl group, chlorine atom, bromine atom, or phenyl group,
29. Any one of the foregoing medicaments, wherein R₁ is methyl group or propyl group,
30. The aforementioned medicament, wherein the active ingredient is selected from the following compounds:
   5-methyl-2-(1-piperazinyl) benzenesulfonic acid;
   5-trifluoromethyl-2-(1-piperazinyl) benzenesulfonic acid;
   5-n-propyl-2-(1-piperazinyl) benzenesulfonic acid;
   5-phenyl-2-(1-piperazinyl) benzenesulfonic acid;
   5-chloro-2-(1-piperazinyl) benzenesulfonic acid;
   5-bromo-2-(1-piperazinyl) benzenesulfonic acid;
   5-isopropyl-2-(1-piperazinyl) benzenesulfonic acid;
   5-cyclohexyl-2-(1-piperazinyl) benzenesulfonic acid;
   5-n-propyl-2-(1-homopiperazinyl) benzenesulfonic acid;
   5-n-propyl-2-[4-(2,3,4-trimethoxybenzyl)-1-piperazinyl] benzenesulfonic acid; and
   5-n-propyl-2-[4-(3,4-dimethoxybenzyl)-1-piperazinyl] benzenesulfonic acid.
31. The aforementioned medicament, wherein the active ingredient is selected from the following compounds:
   5-methyl-2-(1-piperazinyl) benzenesulfonic acid; and
   5-n-propyl-2-(1-piperazinyl) benzenesulfonic acid.
32. Any one of the foregoing medicaments, wherein the active ingredient is 5-methyl-2-(1-piperazinyl) benzenesulfonic acid monohydrate.

### Brief Explanation of the Drawings

Fig. 1 shows Ca leaks from sarcoplasmic reticulum in normal rats and rats with diabetic cardiomyopathy.
Fig. 2 shows an effect of MCC-135 on Ca leaks from sarcoplasmic reticulum in normal rats and rats with diabetic cardiomyopathy.

### Best Mode for Carrying Out the Invention

The present invention will be explained in further detail below.

The present invention provides a medicament which has a therapeutic and /or prophylactic effect for diabetic ischemic heart disease, specifically, heart failure or arrhythmia in diabetic cardiomyopathy. As described above, it is considered that, in diabetic ischemic heart disease such as diabetic cardiomyopathy, a leak of calcium ion from sarcoplasmic reticulum occurs to increase intracellular concentration of free calcium ion, which induces systolic and diastolic dysfunction or arrhythmia. As shown in the examples below, the compound represented by the aforementioned general formula (I) has an inhibitory action on the leak of calcium ion from sarcoplasmic reticulum, and accordingly, has a therapeutic and /or prophylactic effect in heart failure or arrhythmia in a diabetic ischemic heart disease, and in diseases caused by a leak of calcium ion from sarcoplasmic reticulum.

The present invention also provides a medicament which improves a diabetic ischemic heart disease, specifically, cardiac dysfunction in diabetic cardiomyopathy. As described above, when patients with diabetes get ischemic heart disease such as myocardial infarction, a mortality rate significantly increases because of severe heart failure. An exacerbation of cardiac dysfunction, which is derived from reduced action of calcium ion on contractile protein system caused by acidosis, is considered as a cause thereof. As shown in the examples below, the compound represented by the aforementioned general formula (I) blocks, in diabetic cardiomyopathy, the reduced action of calcium ion on a contractile protein system caused by acidosis, and thus has a acidosis-resistant therapeutic and /or prophylactic effect in diabetic ischemic heart disease and diseases caused by cardiac dysfunction, as an agent for improving a cardiac dysfunction in diabetic ischemic diseases.

The active ingredient of the medicament of the present invention includes the aminobenzenesulfonic acid derivative represented by the aforementioned general formula (I) or a salt thereof, or a hydrate thereof or a solvate thereof. Examples of the C₁-C₆ alkyl group defined by R₁ in the aforementioned general formula (I) include methyl group, ethyl group, propyl group, isopropyl group, butyl group, isobutyl group, sec-butyl group, tert-butyl group, pentyl group, isopentyl group, neopentyl group, tert-pentyl group, hexyl group, and isohexyl group. Examples of the C₃-C₇ cycloalkyl group include cyclopropyl group, cyclobutyl group, cyclopentyl group, cyclohexyl group, and cycloheptyl group. Examples of the C₁-C₄ halogenated alkyl group include trifluoromethyl group, trifluoroethyl group, and pentafluoroethyl group. Examples of the halogen atom include fluorine atom, chlorine atom, and bromine atom. Examples of the C₆-C₁₂ aryl group include phenyl group and naphthyl group.

Preferable examples of R₁ include hydrogen atom, a C₁-C₆ alkyl group, a C₅-C₆ cycloalkyl group, trifluoromethyl group, a halogen atom, or phenyl group. More preferable examples of R₁ include a C₁-C₃ alkyl group, a cyclohexyl group, trifluoromethyl group, chlorine atom, bromine atom, or phenyl group. Methyl group or propyl group is particularly preferred.

Examples of the C₁-C₆ alkyl group defined by R₂ include the alkyl groups defined above for R₁. Examples of the C₇-C₁₂ aralkyl group include benzyl group, phenethyl group, and naphthylmethyl group. The aralkyl group may have one or more substituents selected from the group consisting of cyano group; nitro group; a C₁-C₆ alkoxy group such as methoxy group, ethoxy group, propoxy group, isopropoxy group, butoxy group, isobutoxy group, tert-butoxy group, pentyloxy group, isopentyloxy group, tert-pentyloxy group, or hexyloxy group; the halogen atom as defined above for R₁; the alkyl group as defined above for R₁; and amino group.

Preferable examples of R₂ include hydrogen atom, a C₁-C₃ alkyl group, or a C₇-C₁₂ aralkyl group which may have one or more substituents selected from the group consisting of a C₁-C₃ alkyl group, a C₁-C₃ alkoxy group, and a halogen atom. More preferable examples of R₂ include hydrogen atom and a C₇-C₁₂ aralkyl group which may have one or more substituents selected from C₁-C₃ alkoxy groups. Hydrogen atom is particularly preferred.

Further, in the aforementioned general formula (I), n is preferably 2.

Specific preferred examples of the present invention include the compounds shown in the following table 1 and table 2.

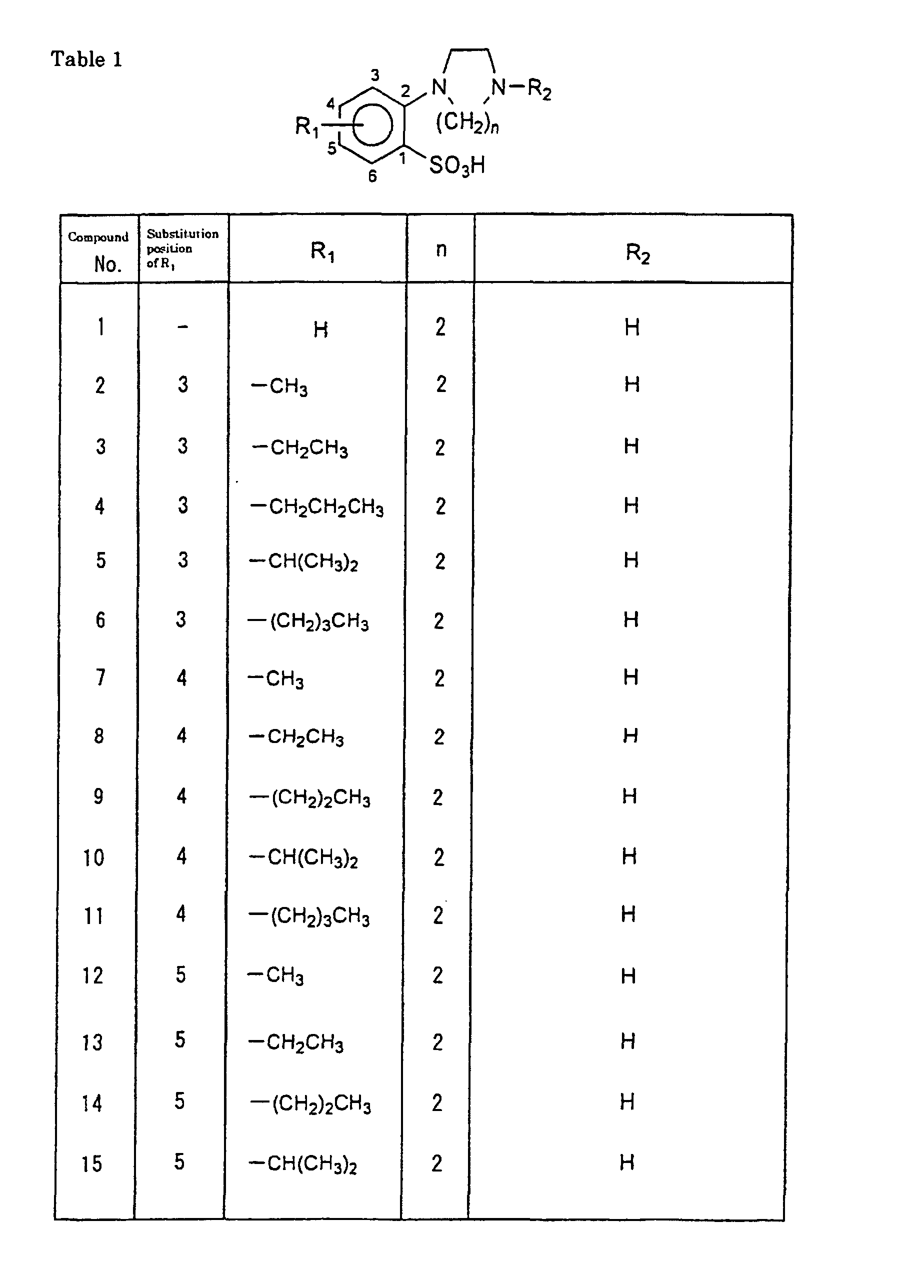

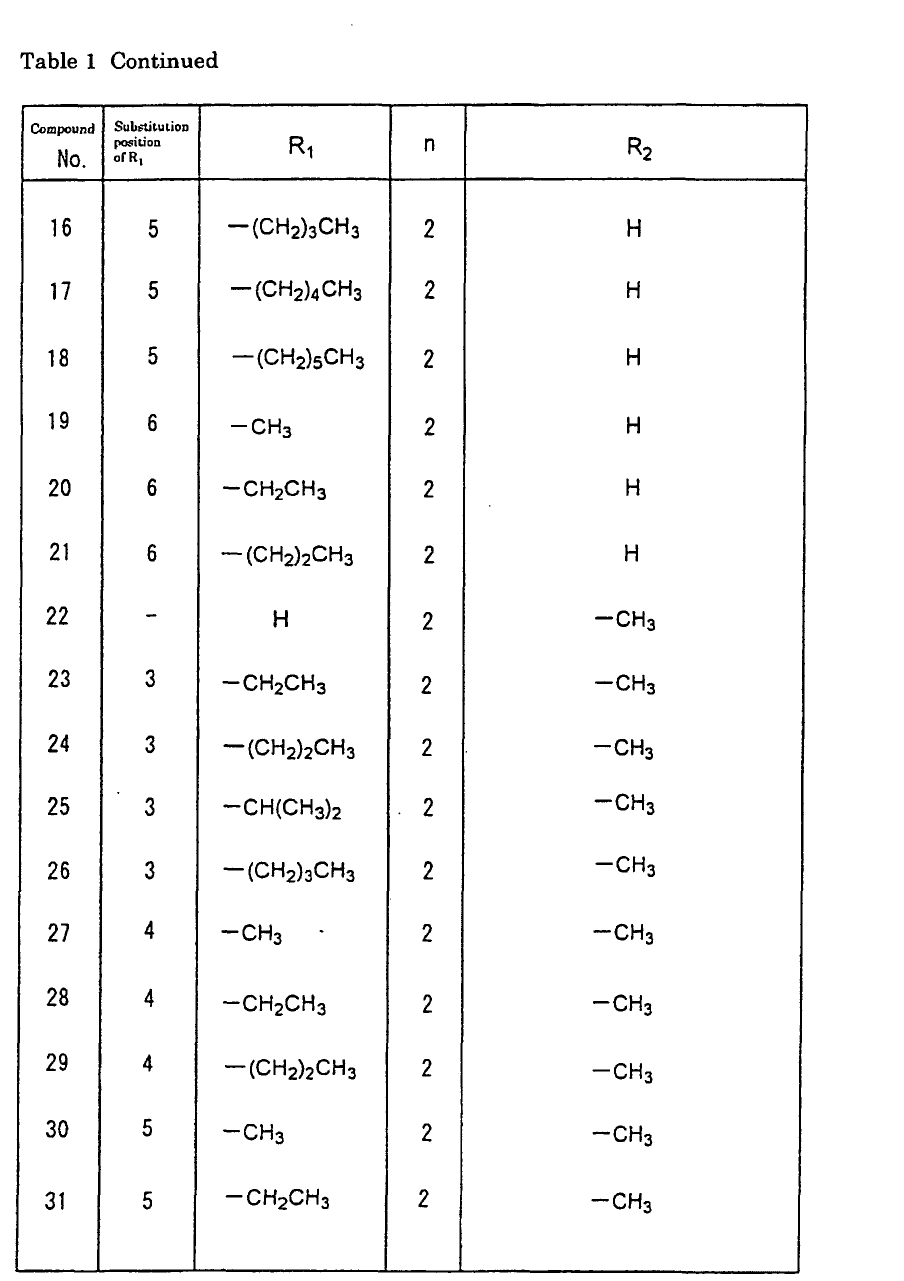

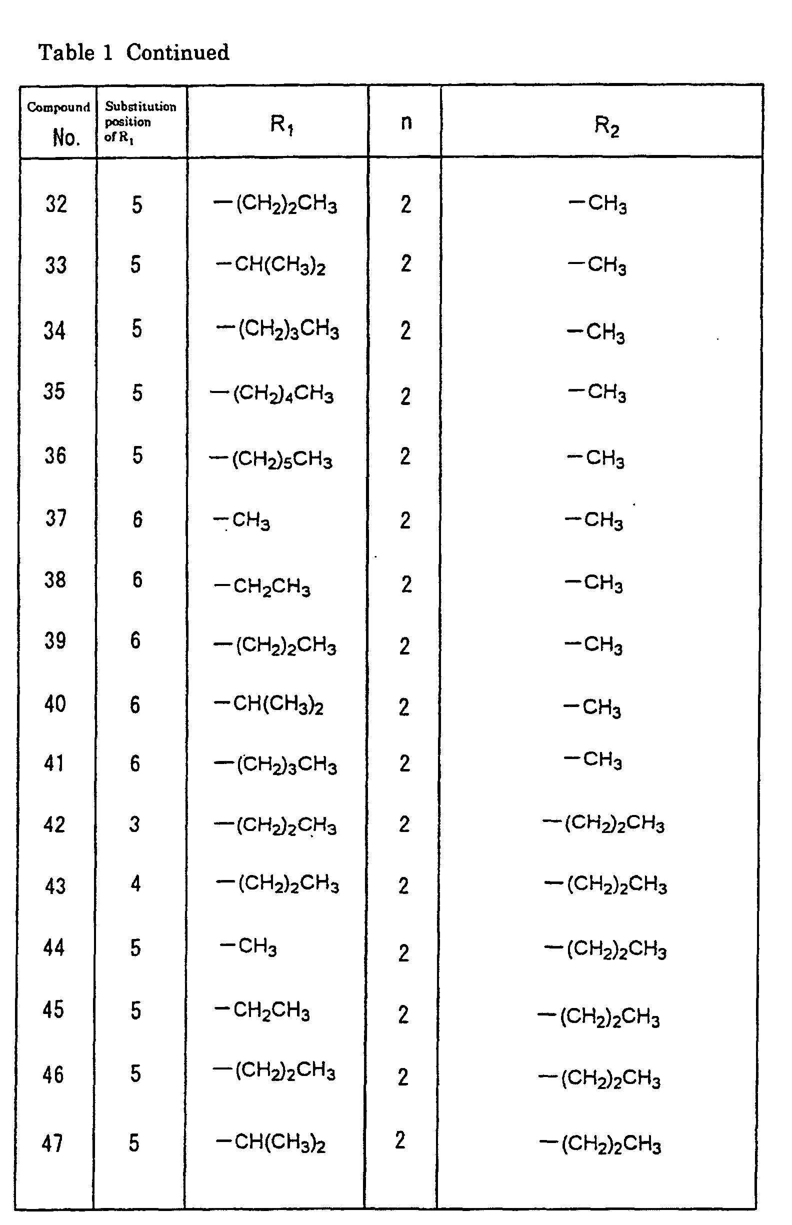

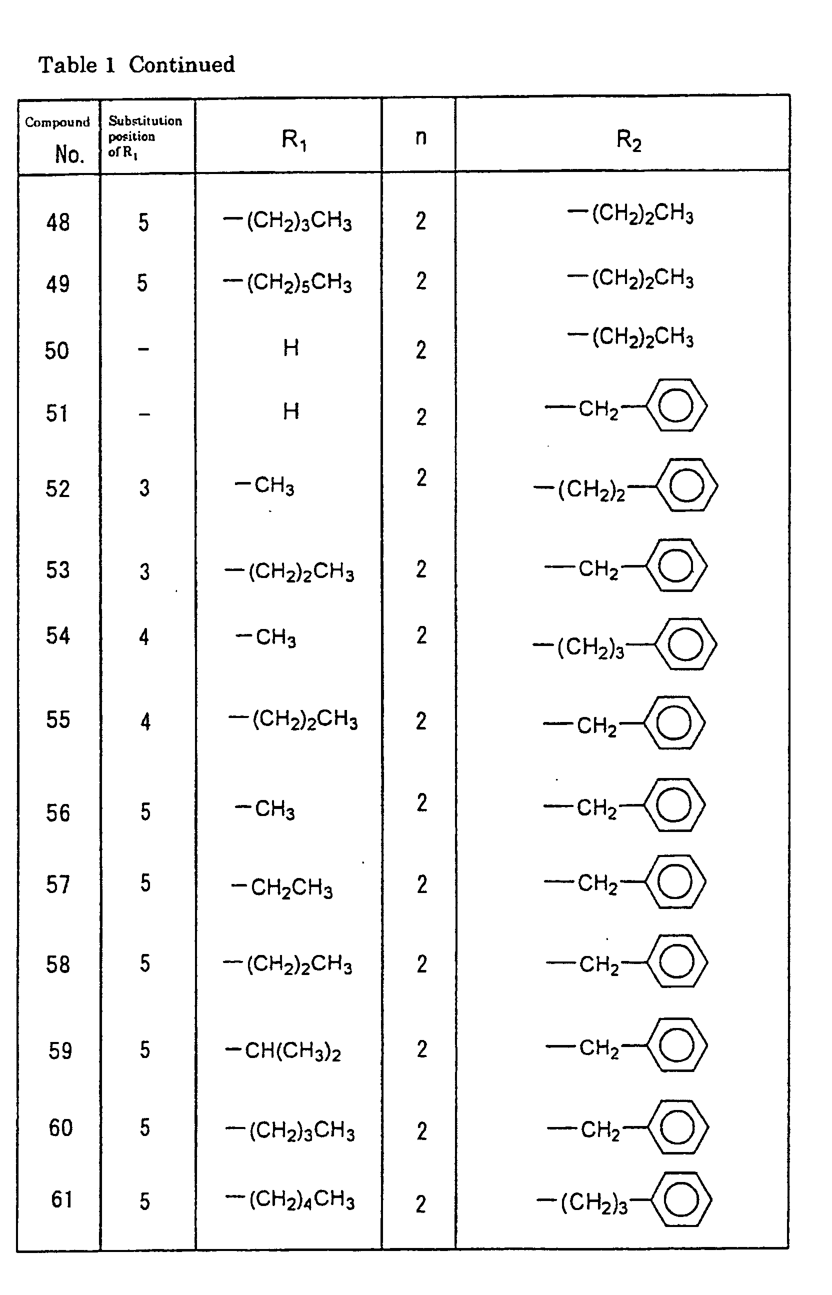

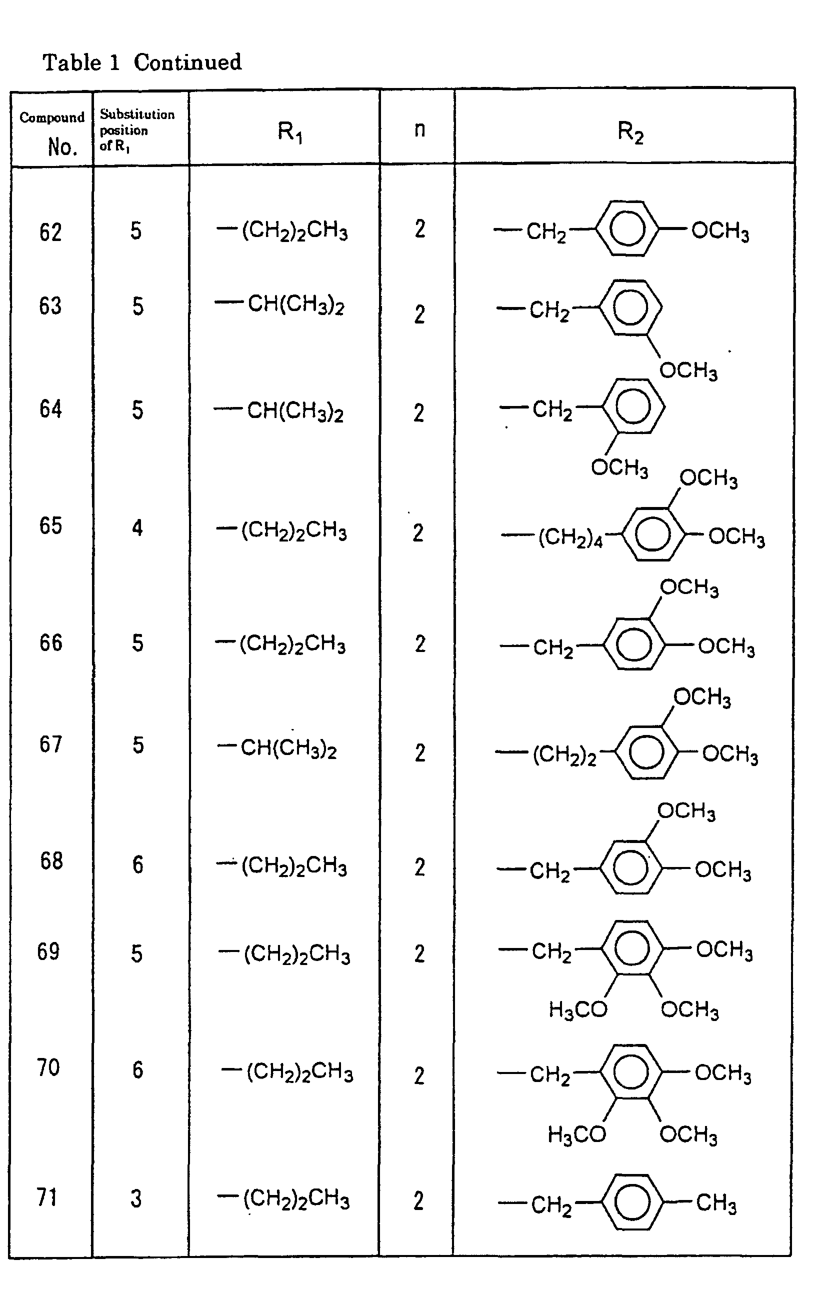

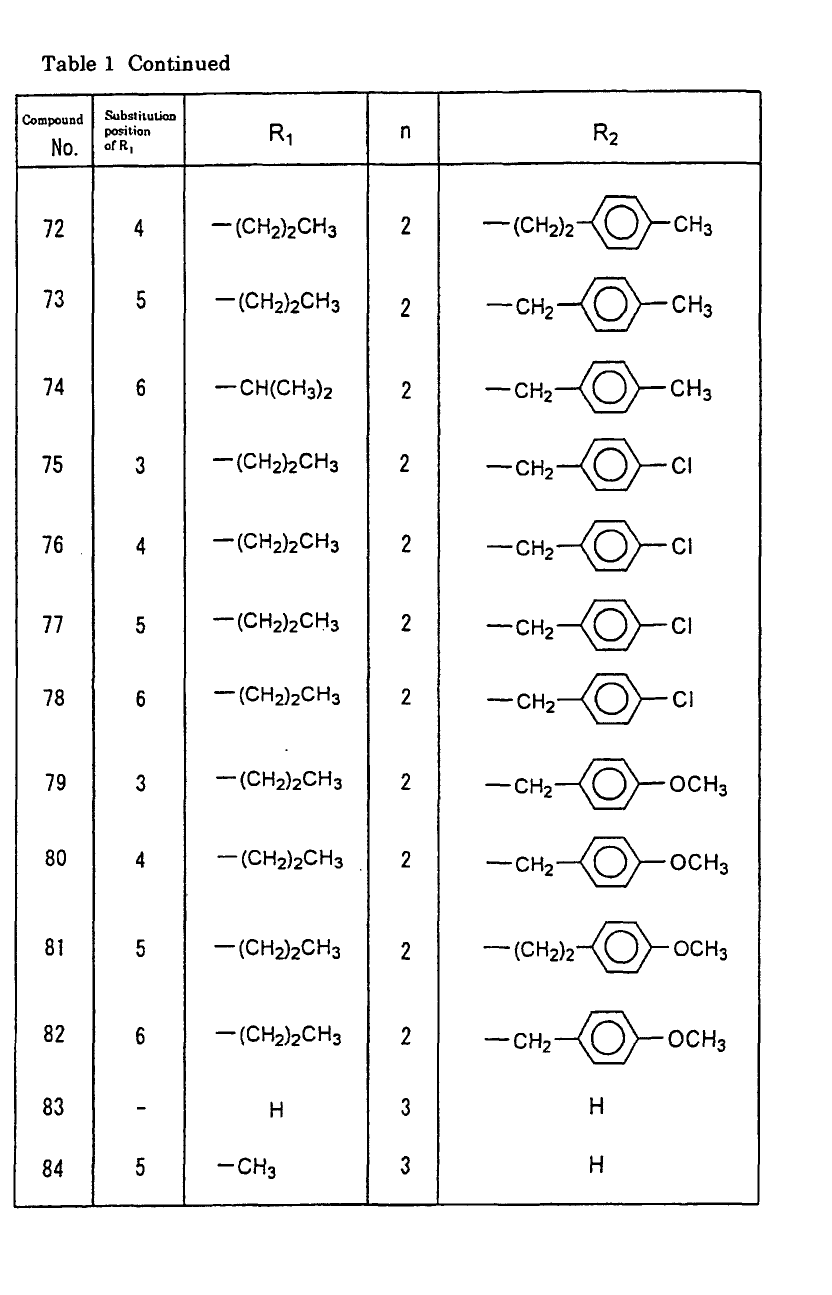

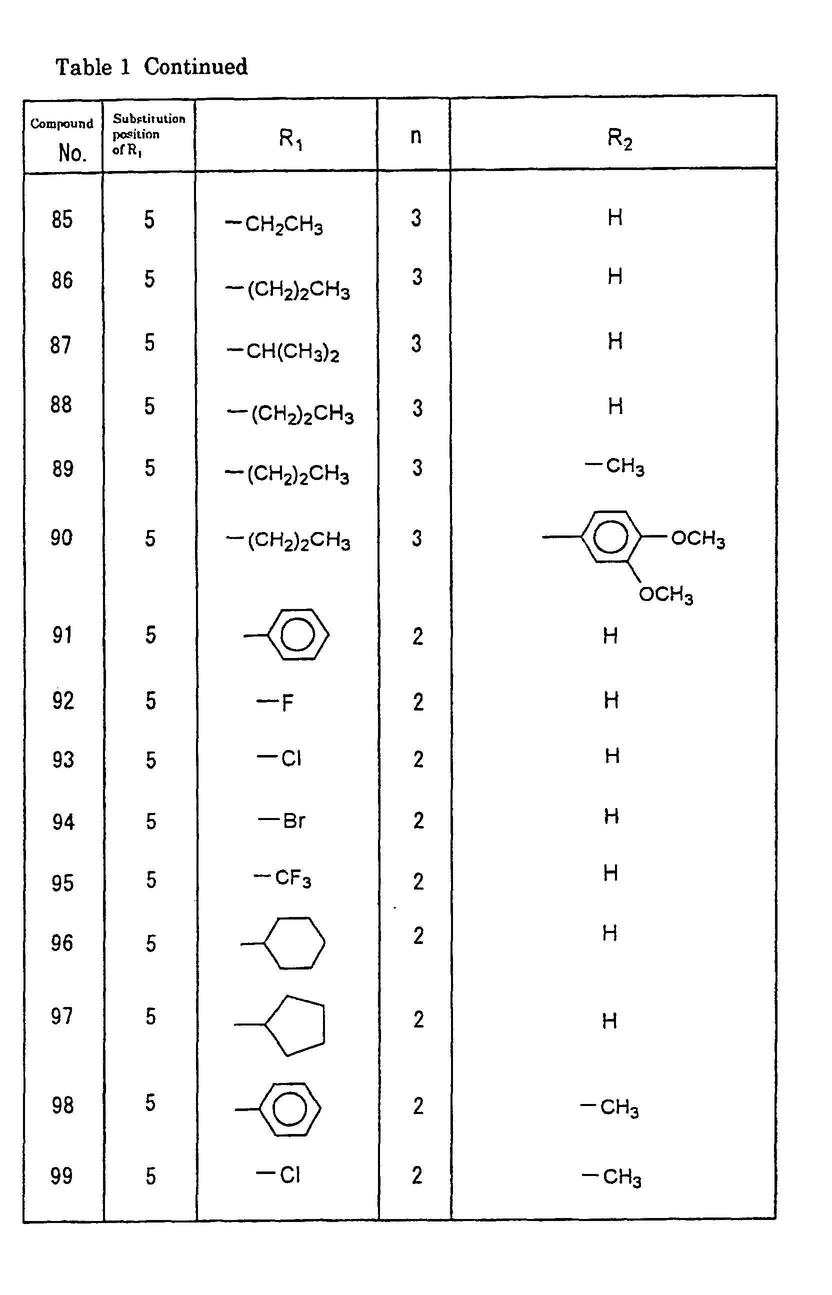

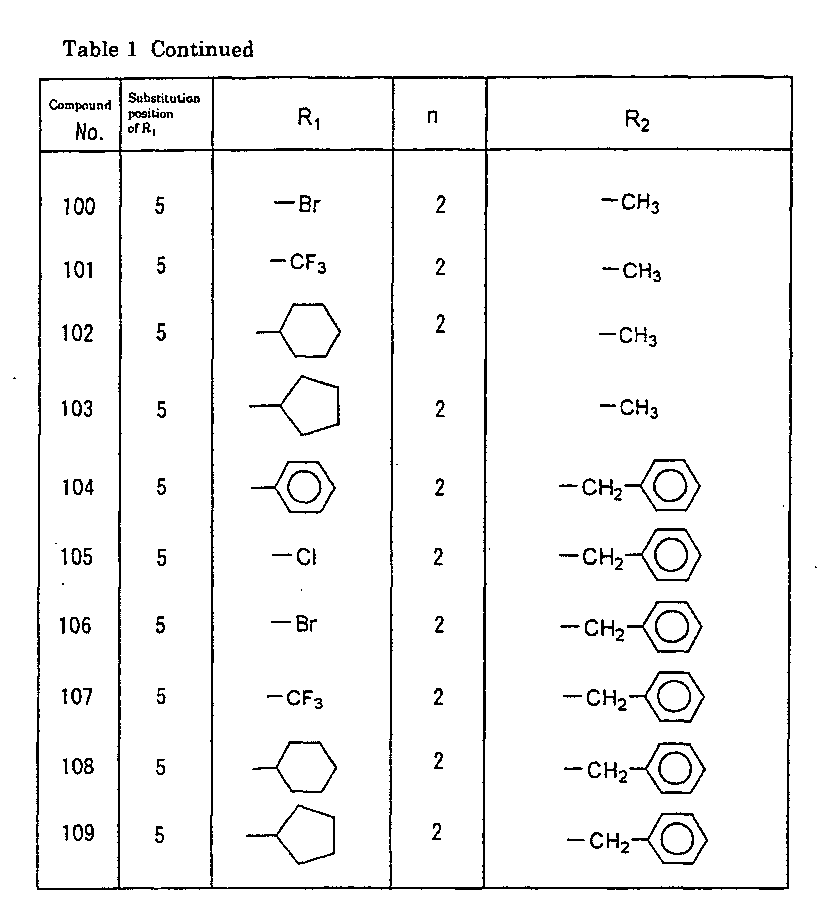

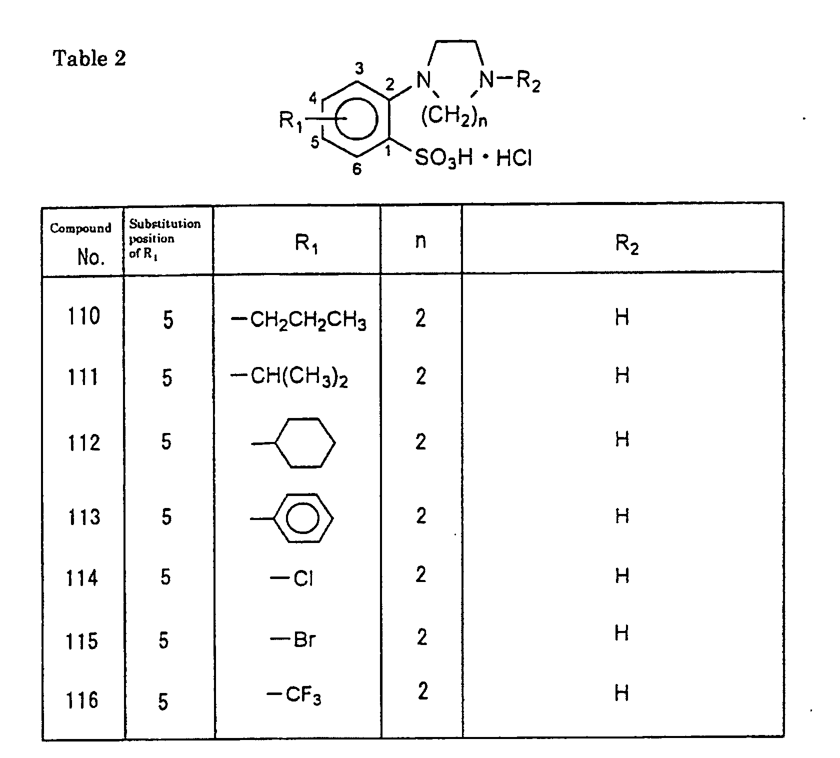

In the table 1 and table 2 above, the compounds wherein the substitution position of R₁ is 5-position is preferable. More preferable examples include the following compounds:
5-methyl-2-(1-piperazinyl) benzenesulfonic acid;
5-trifluoromethyl-2-(1-piperazinyl) benzenesulfonic acid;
5-n-propyl-2-(1-piperazinyl) benzenesulfonic acid;
5-phenyl-2-(1-piperazinyl) benzenesulfonic acid;
5-chloro-2-(1-piperazinyl) benzenesulfonic acid;
5-bromo-2-(1-piperazinyl) benzenesulfonic acid;
5-isopropyl-2-(1-piperazinyl) benzenesulfonic acid;
5-cyclohexyl-2-(1-piperazinyl) benzenesulfonic acid;
5-n-propyl-2-(1-homopiperazinyl) benzenesulfonic acid;
5-n-propyl-2-[4-(2,3,4-trimethoxybenzyl)-1-piperazinyl] benzenesulfonic acid; and
5-n-propyl-2-[4-(3,4-dimethoxybenzyl)-1-piperazinyl] benzenesulfonic acid.

Particularly preferred examples among the above compounds include 5-methyl-2-(1-piperazinyl) benzenesulfonic acid and 5-n-propyl-2-(1-piperazinyl) benzenesulfonic acid.

Physiologically acceptable salts of the above described compounds also fall within the scope of the present invention. Examples of the salts of the above compounds include an alkali metal salt or an alkaline earth metal salt such as sodium salt, potassium salt, magnesium salt, calcium salt, and aluminium salt; an amine salt such as ammonium salt, a lower alkylamine salt such as triethylamine salt, a hydroxy lower alkylamine salt such as 2-hydroxyethylamine salt, bis-(2-hydroxyethyl)amine salt, tris(hydroxymethyl)aminomethane salt, and N-methyl-D-glucamine salt, a cycloalkylamine salt such as dicyclohexylamine salt, a benzylamine salt such as N,N-dibenzylethylenediamine salt, and a dibenzylamine salt; an inorganic acid salt such as hydrochloride, hydrobromide, sulfate, and phosphate; an organic acid salt such as fumarate, succinate, oxalate, and lactate.

Along with the salts and the compounds in free forms, any hydrate or solvate thereof may be used as an active ingredient of the present invention. Examples of solvents that can form the solvate of the aforementioned compound include methanol, ethanol, isopropyl alcohol, acetone, ethyl acetate, and methylene chloride.

A most preferred example of the active ingredient of the present invention includes 5-methyl-2-(1-piperazinyl) benzenesulfonic acid monohydrate.

The aminobenzenesulfonic acid derivative represented by the aforementioned general formula (I) is a known compound, and can be easily prepared by the method described in, for example, Japanese Patent Unexamined Publication (KOKAI) Nos. Hei 3-7263 and Hei 9-221479, European Patent Application Publication Nos. 390,654 and 779,283, U.S. Patent Nos. 5,053,409 and 5,990,113, and thus is easily available to one of ordinary skill in the art.

The agent for therapeutic and /or prophylactic treatment of the present invention can be administered to human orally or parenterally by an ordinary method. Examples of pharmaceutical preparations for oral administration include granules, subtilized granules, powders, tablets, hard capsules, soft capsules, syrups, emulsions, suspensions, and solutions. Examples of pharmaceutical preparations for parenteral administration include injections, suppositories, and transcutaneous preparations.

The active ingredient of the present invention is contained in the aforementioned pharmaceutical preparations together with pharmaceutical carriers which is solid or liquid, or pharmaceutical additives ordinarily used such as excipients, stabilizers, lubricants, sweeteners, preservatives, and suspending agents. A content ratio of the therapeutically or preventively active ingredient based on the carrier ingredient is preferably in a range of from 1 weight % to 90 weight %.

Example of the solid ingredient used include lactose, china clay, sucrose, crystalline cellulose, corn starch, talc, agar, pectin, acacia, stearic acid, magnesium stearate, lecithin, and sodium chloride. Examples of liquid carriers include syrups, glycerol, peanut oil, polyvinylpyrrolidone, olive oil, ethanol, benzyl alcohol, propylene glycol, and water.

A dose of the substance as the active ingredient may be determined suitably for each of the active ingredient, depending on a purpose of therapeutic or prophylactic treatment, a type of a disease to be therapeutically or preventively treated, the condition, body weight, age and sexuality of the patient and the like. As for the compound represented by the aforementioned general formula (I) as a typical example, about 0.01 to 1000 mg for an adult per day may generally be administered orally. The aforementioned dose may desirably be administered once or several times a day as divided portions.

### Examples

The present invention will be explained more specifically by referring to the examples as follows. However, the scope of the present invention is not limited to these examples. As 5-methyl-2-(1-piperazinyl) benzenesulfonic acid monohydrate (hereinafter may sometimes be referred to as "MCC-135") referred to in the following examples, the compound prepared according to the method described in Example 1 in Japanese Patent Unexamined Publication No. Hei 9-221479 was used.

### Example 1

### (Experimental Methods)

Diabetic cardiomyopathy was induced in 7-week old Wistar rats by an intravenous injection of 40 mg/kg of Streptozotocin via each tail (purchased from Sigma, hereinafter may be referred to as "STZ") (J. Exp. Med., 1979, 149: 623-631). Since a severe cardiac dysfunction is observed 5 to 6 months after the administration of STZ (Malone, J.I., Schocken, D.D., Morrison, A.D., Gilbert-Barness, E., Diabetic cardiomyopathy and carnitine deficiency. J-Diabetes-Complications. 1999, 13(2): 86-90), the rats were subjected to the experiments 7 months after the administration of STZ. The rats were sacrificed and the hearts of the rats were isolated 7 months after the administration of STZ. The following experiment was conducted according to the method of Kitada et al. (J. Pharmacol. Exp. Ther. 1987, 243: 633-638). Skinned fiber preparations were prepared by saponin treatments of papillary muscles of the hearts isolated from the rats 7 months after the administration of STZ and the control rats. A medium for the preparations was changed from Ca (calcium ion) free buffer solution to a solution of pCa 6.7 to have sarcoplasmic reticulum uptake a certain amount of Ca in the presence of ATP. Then, the medium was changed to a buffer containing 5-methyl-2-(1-piperazinyl) benzenesulfonic acid monohydrate (hereinafter may be referred to as "MCC-135") or vehicle for the drug and the preparations were left stand for two minutes to allow Ca leakage from sarcoplasmic reticulum during the period. Then all of the remaining Ca in the sarcoplasmic reticulum was released by using 50 mM caffeine. A tension generated was used as an index, and a difference of the tension between the group administered with MCC-135 and the control group was examined.

### (Results)

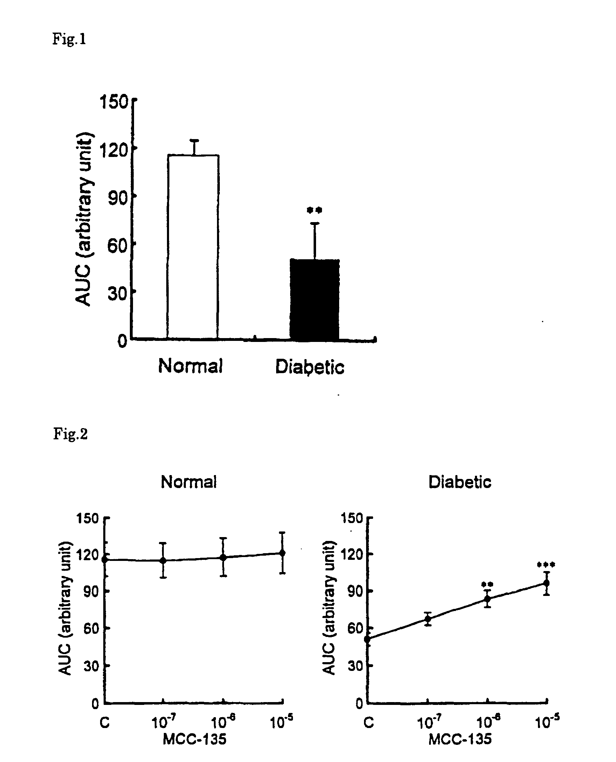

Fig. 1 shows each Ca leakage from sarcoplasmic reticulum in normal rats ("Normal" in Fig. 1) and rats with diabetic cardiomyopathy ("Diabetic" in Fig. 1) (N=7 in both of them). P value obtained by t-test for the rats with diabetic cardiomyopathy versus the normal rats was less than 0.01 (** in Fig. 1). As shown in Fig. 1, a significant Ca leakage from sarcoplasmic reticulum was observed in the diabetic cardiomyopathy group.

Fig. 2 shows an effect of MCC-135 on the Ca leakage from sarcoplasmic reticulum in the normal rats ("Normal" in the left side of Fig. 2) and the rats with diabetic cardiomyopathy ("Diabetic" in the right side of Fig. 2) (N =7 in both of them). In the figure, P values obtained by one way ANOVA analysis and Dunnett test for the rats with diabetic cardiomyopathy versus the controls were less than 0.01 in the group administered with 10⁻⁶ M of MCC-135 (** in Fig. 2), and less than 0.001 in the group administered with 10⁻⁵ M of MCC-135 (** in Fig. 2). No Ca leakage occured in the sarcoplasmic reticulum of the normal rats, and under these condition, MCC-135 (the dose: 10⁻⁷ M to 10⁻⁵ M) had no effect. Whilst, MCC-135 inhibited Ca leakage from the sarcoplasmic reticulum with diabetic cardiomyopathy in a concentration-dependant manner.

### (Conclusion)

A Ca leakage from sarcoplasmic reticulum increases in diabetic cardiomyopathy, which is considered to be a cause of heart failure or arrhythmia. Diabetic cardiomyopathy is resistant to conventional therapeutic medicaments. A lack of direct inhibitory effect of the conventional medicaments on Ca leakage from sarcoplasmic reticulum is considered to be a reason of the resistance, and as a result, they presumably are not clinically effective. As described above, MCC-135 inhibited Ca leakage from sarcoplasmic reticulum in a concentration-dependant manner, which clearly demonstrates that MCC-135 can be used as a new-type therapeutic and/or prophylactic medicament which has not been available so far.

### Example 2

### (Methods)

Diabetic cardiomyopathy model is prepared in a similar manner to that in Example 1. The rats 7 months after the administration of STZ were subjected to the experiments in a similar manner to that in Example 1. The rats were sacrificed and the hearts of the rats were isolated 7 months after the administration of STZ. The following experiment was conducted according to the method of Kitada et al. which is the same as that in the Example 1. Left ventricular papillary muscles were cut off from the isolated heart in an ice cooled Krebs-Henseleit solution, and contraction-relaxation function of the papillary muscles was measured by Magnus method. Some of the papillary muscles were applied to a functional analysis on a relation between various calcium ion (Ca) concentrations and contractile force by skinned fiber method with saponin treatment. Acidosis was induced by lowering the pH of the solution from 7.0 in normal to 6.6. A mode of change of relation between the various calcium ion (Ca) concentrations and the contractile forces in the presence or absence of MCC-135 was studied.

### (Results)

Table 3 shows the blood glucose level of the STZ model. Apparently, the glucose level in the blood increased significantly by the administration of STZ (approximately 4 fold increase). In addition, a decrease of the body weight, cardiac hypertrophy (an increase of the ratio of: the left ventricular weight /the body weight), and pulmonary congestion (an increase of the ratio of: the lung weight/the body weight) were observed. These changes are similar to the pathological condition of cardiomyopathy and heart failure. In Table 1, ** represents that the value P obtained by t-test versus the normal rats is less than 0.01, and *** represents less than 0.001.

**Table 3**

| Blood glucose level of the STZ model animal | | |
|---|---|---|
| | Normal rat (n=7) | Rat with cardiomyopathy (n=7) |
| Plasma glucose level (mg/dL) | 164±10 | 636±32 *** |
| Body weight (g) | 424±8 | 130±9 *** |
| Weight of the left ventricular (mg) | 684±8 | 373±24 *** |
| Weight of the right ventricular (mg) | 159±5 | 89±4 *** |
| Weight of lung (mg) | 1.46±0.05 | 1.13±0.10 ** |
| The left ventricular weight/body weight (×10⁻³) | 1.62±0.03 | 2.88±0.08 *** |
| The right ventricular weight/body weight (×10⁻⁴) | 3.74±0.06 | 6.94±0.26 *** |
| Lung/body weight (×10⁻³) | 3.46±0.15 | 8.71±0.57 *** |

Table 4 shows effect of acidosis and the action of MCC-135 on cardiac muscles of the normal rats and the rats with diabetic cardiomyopathy. In the normal heart, a decrease in Ca sensitivity (pCa50 in Table 4) and a decrease in cross bridge interaction (Maximum Force in Table 4) by acidosis were observed. The decrease in the cross bridge interaction by acidosis observed in the normal heart was enhanced in the diabetic cardiomyopathy. In the diabetic cardiomyopathy, resting tension ("Resting force" in Table 4) was increased, which was inhibited by acidosis. MCC-135 had no effect on the action of acidosis on the normal hearts, whilst MCC-135 blocked the action of acidosis in the diabetic cardiomyopathy. In Table 4, Hill n represents Hill coefficient (cooperativity of Ca and contractile protein system), * represents that the value P is less than 0.05 versus the control, ** represents that the value P is less than 0.01 versus the control, *** represents that the value P is less than 0.01 versus acidosis, and **** represents that P is less than 0.01 versus the control in the normal rats.

**Table 4**

| Comparison of various parameters in normal rats and rats with diabetic cardiomyopathy | | | | |
|---|---|---|---|---|
| | PCa50 | Hill n | Maximum Force (mN/mm²) | Resting Force (mN/mm²) |
| Normal rat Control (pH7.0) | 5.50±0.01 | 2.21±0.11 | 0.453±0.037 | 0±0 |
| Normal rat Acidosis (pH6.6) | 5.43±0.01 ** | 3.19±0.45 ** | 0.377±0.021 ** | 0±0 n.s |
| Normal rat Acidosis (pH6.6) +1µM MCC-135 | 5.43±0.01 ** | 3.17±0.42 ** | 0.371±0.027 ** | 0±0 n.s |
| Rat with cardiomyopathy Control (pH7.0) | 5.54±0.03 **** | 2.07±0.09 | 0.328±0.019 **** | 0.004±0.000 **** |
| Rat with cardiomyopathy Acidosis (pH6.6) | 5.58±0.05 n.s | 2.23±0.41 n.s | 0.249±0.019 ** | 0.002±0.002 * |
| Rat with cardiomyopathy Acidosis (pH6.6) +1µM MCC-135 | 5.55±0.07 n.s | 2.12±0.20 n.s | 0.286±0.020 **, *** | 0.000±0.000 **, *** |

### (Conclusion)

Exacerbation of a contractile dysfunction by acidosis was observed in diabetic cardiomyopathy. A decrease in responsiveness of contractile protein system itself to calcium is important as a cause of the exacerbation. The above result indicates that MCC-135 inhibits the exacerbation caused by acidosis. Accordingly, it can be understood that MCC-135 improves a cardiac function by directly acting on the decrease in the cross bridge interaction caused by acidosis. Effectiveness for improvement of a cardiac function of diabetic cardiomyopathy can not be expected for conventional medicaments which have an action of increasing intracellular Ca concentration. Therefore, as shown in the results above, it is apparent that MCC-135, which blocks the decrease in responsiveness of contractile protein system itself to calcium, can be used as a new-type of medicament for therapeutic and /or prophylactic treatment of diabetic heart diseases, which has not been available so far.

### Industrial Applicability

According to the present invention, a new type of medicament having acidosis-resistant improving effect on a cardiac function can be provided, which has not been known in conventional medicaments used for diabetic ischemic heart disease. The medicament of the present invention is useful as an agent for therapeutic and /or prophylactic treatment of diabetic ischemic heart disease.

The present application was filed with claiming the priorities on the basis of the Japanese Patent Application Nos.2001-69552 and 2001-69553.

## Claims

1. An agent for therapeutic and /or prophylactic treatment of heart failure or arrhythmia in diabetic ischemic heart disease, which comprises as an active ingredient an aminobenzenesulfonic acid derivative represented by the following general formula (I) 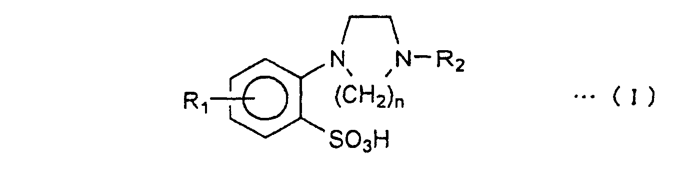 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.

2. The agent for therapeutic and /or prophylactic treatment according to claim 1, **characterized in that** the diabetic ischemic heart disease is diabetic cardiomyopathy.

3. The agent for therapeutic and /or prophylactic treatment according to claim 1 or 2, **characterized in that** said agent is based on an inhibition of a leak of calcium ion from sarcoplasmic reticulum in the diabetic ischemic heart disease.

4. An agent for therapeutic and /or prophylactic treatment of a disease caused by a leak of calcium ion from sarcoplasmic reticulum, which comprises as an active ingredient an aminobenzenesulfonic acid derivative represented by the following general formula (I) 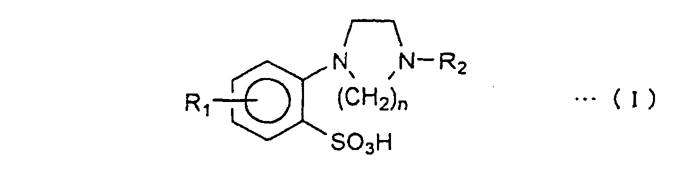 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.

5. An agent for therapeutic and /or prophylactic treatment of diabetic ischemic heart disease, **characterized in that** said agent improves a cardiac dysfunction in diabetic ischemic heart disease, and comprises as an active ingredient an aminobenzenesulfonic acid derivative represented by the following general formula (I) 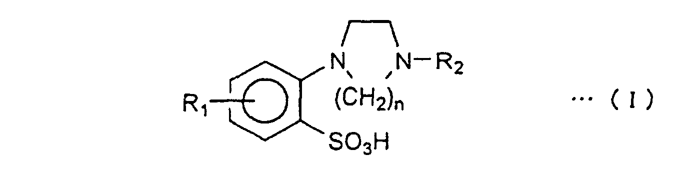 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.

6. The agent for therapeutic and /or prophylactic treatment according to claim 5, **characterized in that** the diabetic ischemic heart disease is diabetic cardiomyopathy.

7. The agent for therapeutic and /or prophylactic treatment according to claim 5 or 6, **characterized in that** the cardiac dysfunction is cardiac dysfunction resistant to acidosis.

8. An agent for therapeutic and /or prophylactic treatment of a disease caused by a cardiac dysfunction resistant to acidosis, which comprises as an active ingredient an aminobenzenesulfonic acid derivative represented by the following general formula (I) 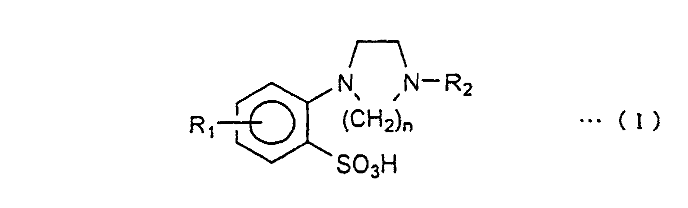 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.

9. The agent for therapeutic and /or prophylactic treatment according to any one of claims 1 to 8, wherein the substitution position of R₁ is 5-position.

10. The agent for therapeutic and /or prophylactic treatment according to any one of claims 1 to 9, wherein n is 2.

11. The agent for therapeutic and /or prophylactic treatment according to any one of claims 1 to 10, wherein R₂ is hydrogen atom, a C₁-C₃ alkyl group, or a C₇-C₁₂ aralkyl group which may have one or more substituents selected from the group consisting of a C₁-C₃ alkyl group, a C₁-C₃ alkoxy group, and a halogen atom.

12. The agent for therapeutic and /or prophylactic treatment according to any one of claims 1 to 11, wherein R₂ is hydrogen atom or a C₇-C₁₂ aralkyl group which may have one or more substituents selected from the group consisting of a C₁-C₃ alkoxy group.

13. The agent for therapeutic and /or prophylactic treatment according to any one of claims 1 to 12, wherein R₂ is hydrogen atom.

14. The agent for therapeutic and /or prophylactic treatment according to any one of claims 1 to 13, wherein R₁ is hydrogen atom, a C₁-C₆ alkyl group, a C₅-C₆ cycloalkyl group, trifluoromethyl group, a halogen atom, or phenyl group.

15. The agent for therapeutic and /or prophylactic treatment according to any one of claims 1 to 14, wherein R₁ is a C₁-C₃ alkyl group, cyclohexyl group, trifluoromethyl group, chlorine atom, bromine atom, or phenyl group.

16. The agent for therapeutic and /or prophylactic treatment according to any one of claims 1 to 15, wherein R₁ is methyl group or propyl group.

17. The agent for therapeutic and /or prophylactic treatment according to any one of claims 1 to 8, wherein the active ingredient is selected from the following compounds:
5-methyl-2-(1-piperazinyl) benzenesulfonic acid;
5-trifluoromethyl-2-(1-piperazinyl) benzenesulfonic acid;
5-n-propyl-2-(1-piperazinyl) benzenesulfonic acid;
5-phenyl-2-(1-piperazinyl) benzenesulfonic acid;
5-chloro-2-(1-piperazinyl) benzenesulfonic acid;
5-bromo-2-(1-piperazinyl) benzenesulfonic acid;
5-isopropyl-2-(1-piperazinyl) benzenesulfonic acid;
5-cyclohexyl-2-(1-piperazinyl) benzenesulfonic acid;
5-n-propyl-2-(1-homopiperazinyl) benzenesulfonic acid;
5-n-propyl-2-[4-(2,3,4-trimethoxybenzyl)-1-piperazinyl] benzenesulfonic acid; and
5-n-propyl-2-[4-(3,4-dimethoxybenzyl)-1-piperazinyl] benzenesulfonic acid.

18. The agent for therapeutic and /or prophylactic treatment according to claim 17, wherein the active ingredient is selected from the following compounds:
5-methyl-2-(1-piperazinyl) benzenesulfonic acid; and
5-n-propyl-2-(1-piperazinyl) benzenesulfonic acid.

19. The agent for therapeutic and /or prophylactic treatment according to any one of claims 1 to 18, wherein the active ingredient is 5-methyl-2-( 1-piperazinyl) benzenesulfonic acid monohydrate.

20. An inhibitor of a leak of calcium ion from sarcoplasmic reticulum in diabetic ischemic heart disease, which comprises as an active ingredient an aminobenzenesulfonic acid derivative represented by the following general formula (I) 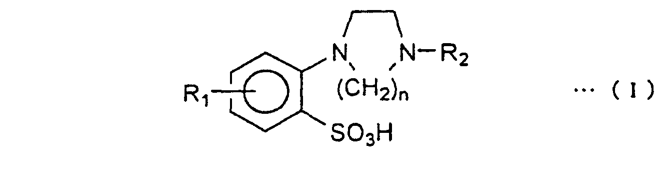 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.

21. An agent for acidosis-resistant improvement of cardiac dysfunction in diabetic ischemic heart disease, which comprises as an active ingredient an aminobenzenesulfonic acid derivative represented by the following general formula (I) 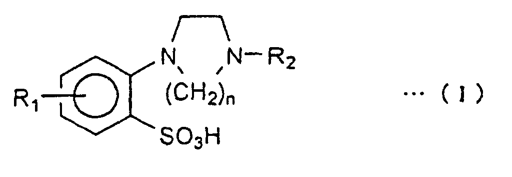 (wherein R₁ represents hydrogen atom, a C₁-C₆ alkyl group, a C₃-C₇ cycloalkyl group, a C₁-C₄ halogenated alkyl group, a halogen atom, or a C₆-C₁₂ aryl group; R₂ represents hydrogen atom, a C₁-C₆ alkyl group, or a C₇-C₁₂ aralkyl group which may have one or more substituents selected from the group consisting of cyano group, nitro group, a C₁-C₆ alkoxy group, a halogen atom, a C₁-C₆ alkyl group, and amino group; and n represents an integer of from 1 to 4) or a salt thereof, or a hydrate thereof or a solvate thereof.

22. The medicament according to claim 20 or 21, wherein the substitution position of R₁ is 5-position.

23. The medicament according to any one of claims 20 to 22, wherein n is 2.

24. The medicament according to any one of claims 20 to 23, wherein R₂ is hydrogen atom, a C₁-C₃ alkyl group, or a C₇-C₁₂ aralkyl group which may have one or more substituents selected from the group consisting of a C₁-C₃ alkyl group, a C₁-C₃ alkoxy group, and a halogen atom.

25. The medicament according to any one of claims 20 to 24, wherein R₂ is hydrogen atom, or a C₇-C₁₂ aralkyl group which may have one or more substituents selected from the group consisting of a C₁-C₃ alkoxy group.

26. The medicament according to any one of claims 20 to 25, wherein R₂ is hydrogen atom.

27. The medicament according to any one of claims 20 to 26, wherein R₁ is hydrogen atom, a C₁-C₆ alkyl group, a C₅-C₆ cycloalkyl group, trifluoromethyl group, a halogen atom, or phenyl group,

28. The medicament according to any one of claims 20 to 27, wherein R₁ is a C₁-C₃ alkyl group, cyclohexyl group, trifluoromethyl group, chlorine atom, bromine atom, or phenyl group,

29. The medicament according to any one of claims 20 to 28, wherein R₁ is methyl group or propyl group,

30. The medicament according to claim 20 or 21, wherein the active ingredient is selected from the following compounds:
5-methyl-2-(1-piperazinyl) benzenesulfonic acid;
5-trifluoromethyl-2-(1-piperazinyl) benzenesulfonic acid;
5-n-propyl-2-(1-piperazinyl) benzenesulfonic acid;
5-phenyl-2-(1-piperazinyl) benzenesulfonic acid;
5-chloro-2-(1-piperazinyl) benzenesulfonic acid;
5-bromo-2-(1-piperazinyl) benzenesulfonic acid;
5-isopropyl-2-(1-piperazinyl) benzenesulfonic acid;
5-cyclohexyl-2-(1-piperazinyl) benzenesulfonic acid;
5-n-propyl-2-(1-homopiperazinyl) benzenesulfonic acid;
5-n-propyl-2-[4-(2,3,4-trimethoxybenzyl)-1-piperazinyl] benzenesulfonic acid; and
5-n-propyl-2-[4-(3,4-dimethoxybenzyl)-1-piperazinyl] benzenesulfonic acid.

31. The medicament according to claim 30, wherein the active ingredient is selected from the following compounds:
5-methyl-2-(1-piperazinyl) benzenesulfonic acid; and
5-n-propyl-2-(1-piperazinyl) benzenesulfonic acid.

32. The medicament according to any one of claims 20 to 31, wherein the active ingredient is 5-methyl-2-(1-piperazinyl) benzenesulfonic acid monohydrate.
